# EUROPÄISCHE PATENTSCHRIFT

(11) **EP 0 375 600 B1**
(45) Veröffentlichungstag und Bekanntmachung des Hinweises auf die Patenterteilung: **19.10.1994**
(21) Anmeldenummer: 89730226.1
(22) Anmeldetag: 20.12.1989
(51) Int. Cl.: A61F 2/30, A61F 2/32, C23C 14/00, B23K 15/00

(54) **Verfahren zur Herstellung einer Endoprothese**
Method for producing an endoprosthesis
Méthode de formation d'une endoprothèse

(30) Priorität: 23.12.1988 DE 3844155
(43) Veröffentlichungstag der Anmeldung: 27.06.1990
(73) Patentinhaber: Johnson & Johnson Professional Products GmbH, 22848 Norderstedt (DE)
(72) Erfinder: Kranz, Curt, Dr., D-1000 Berlin 62 (DE)
(74) Vertreter: UEXKÜLL & STOLBERG

(56) Entgegenhaltungen:
- DE-A- 3 715 000
- DE-A- 3 716 026
- US-A- 4 687 675

## Beschreibung

Die Erfindung betrifft eine Endoprothese der im Oberbegriff des Anspruchs 1 angegebenen Art sowie entsprechende Herstellungsverfahren.

Hohlschaftendoprothesen aus Metallegierungen wurden bisher vorrangig als Gußteile hergestellt. Diese Technologie ist jedoch auf bestimmte Materialien beschränkt. Die vorrangig verwendeten gießbaren Werkstoffe für Prothesenschäfte sind bisher eine Kobalt-Chrom-Molybdän-Gußlegierung sowie 316-Gußstahl.

Nachteilig ist dabei, daß diese Werkstoffe nur eine geringe Ermüdungsfestigkeit aufweisen, so daß Ermüdungsbrüche aufgrund erhöhter Biegewechselbeanspruchung auftreten können. Ein anderer Werkstoff, die Titan-Aluminium-Vanadium-Schmiedelegierung wird bisher ausschließlich bei Vollschaftprothesen genutzt.

Eine Endoprothese der eingangs genannten Gattung ist aus der DE-A-3 715 000 bekannt. Hierbei sind einzelne erforderlichenfalls vorgeformte gelochte Blechstreifen durch Schweißen zu einem Prothesenhohlschaftes miteinander verbunden.

Hierbei ist nachteilig, daß durch die Formgebung aus - gewöhnlich zunächst eine konstante Materialstärke aufweisendem Blech - umfangreiche Nachbearbeitungen notwendig sind. Sämtliche Anpassungen der Biegesteifigkeit können nur durch örtliche Materialabtragung erfolgen. Die Blechdicke kann - auch wenn es rechnerisch erforderlich wäre - in keinem Fall nachträglich heraufgesetzt werden. Wird die Blechstärke aber von vorn herein so hoch gewählt, daß allein durch Materialabtrag jede gewünschte örtliche Wandungsdicke erzeugt werden kann, so macht die dann erforderliche Ausgangsmaterialstärke die Herstellung vom Materialbedarf her kostspielig und insbesondere auch die Verformung aufwendig.

Die Wandstärke der Blechstreifen wird an geeigneten Stellen durch das Vorsehen von unterschiedlich dimensionierten Löchern sowie durch Fräsen oder Schleifen verringert. Dadurch soll die Biegesteifigkeit des Prothesenhohlschaftes besser der Biegesteifigkeit des umgebenden Knochens entsprechen.

Der Erfindung liegt die Aufgabe zugrunde, eine Hohlschaftendoprothese der eingangs genannte Art anzugeben, welche wirtschaftlich herstellbar und dabei eine große Freiheit bei der Formgebung sowohl im Hinblick auf ihre äußeren als auch auf ihre inneren Wandungsbereiche zuläßt und mit der gleichzeitig eine gute Anpassung der Biege- und Längssteifigkeit der Endoprothese an die Biege- und Längssteifigkeit des umgebenden Knochens möglich ist sowie ein Verfahren zu ihrer Herstellung auzugeben.

Diese Aufgabe wird mit den kennzeichnenden Merkmalen des Anspruchs 1 sowie durch das Verfahren mit den kennzeichnenden Merkmalen des Anspruchs 9 gelöst.

Die Erfindung beruht auf der Erkenntnis, daß die bevorzugt verwendbare Titan- Aluminium-Vanadium-Legierung durch bekannte Preß- oder Schmiedeverfahren in beliebige Rohteile formbar ist und daß diese Rohteile mittels Schweißverfahren zusammenfügbar sind. Besonders vorteilhaft ist dabei die unkomplizierte und bequeme Bearbeitbarkeit der Rohteile vor dem Zusammenschweißen, die sich wesentlich einfacher gestaltet als ein Nachbearbeiten eines gegossenen Hohlschaftes oder eine Verwendung eines komplizierten Gußkernes. Dabei weisen die Rohteile die Form von zwei Halbschalen auf. Diese Halbschalen werden, soweit möglich, bereits mit kontinuierlicher Dickenabnahme in Richtung von gelenknah nach gelenkfern gepreßt oder geschmiedet. Um die technischen Grenzen des Schmiedeverfahrens in Form einer Mindestdicke des zu bearbeitenden Materials auszugleichen, erweist sich ein Nachbearbeiten, vorzugsweise durch chemisches Abtragen als günstig.

Um eine lokale Veränderung der Biege- und/oder Längssteifigkeit zu bewirken, kann eine gezielte Schwächung der Schaftwandung vorgesehen werden.

Auch Durchbrüche in den Wandungen der Halbschalen können bereits beim Pressen oder Schmieden berücksichtigt werden. Eventuelle Grate lassen sich aufgrund der Zweistufigkeit des erfindungsgemäßen Verfahrens schnell und problemlos noch vor dem Zusammenfügen der beiden Einzelteile entfernen.

Ebenso können auch andere Abweichungen von einer regelmäßigen Form, wie zum Beispiel Vertiefungen in der konkaven oder der konvexen Oberfläche der Halbschalen durch das Preß- oder Schmiedeverfahren zumindest vorgeformt werden.

Es wurde ferner gefunden, daß insbesondere die Titan-Aluminium-Vanadium-Schmiedelegierung (TiAl6V4) gute Biokompatibilität, hohe Ermüdungsfestigkeit und gute Korrosionsbeständigkeit besitzt und daher als Werkstoff zur Herstellung von Prothesenschäften besonders geeignet ist. Die Verarbeitung dieses Materials mittels Schmiede- oder Preß- und Schweißverfahren ist wirtschaftlich, da nur einfache Formen erforderlich sind und der Herstellungszeitaufwand pro Stück gering ist.

Das Zusammenfügen der beiden Halbschalen erfolgt, einem vorteilhaften Verfahren zur Herstellung der erfindungsgemäßen Endoprothese gemäß, durch Elektronenstrahlschweißen. Dieses Schweißverfahren zeichnet sich vor allem durch sehr schmale und gleichmäßige Schweißnähte aus und genügt demzufolge den hohen Qualitätsanforderungen, die an Prothesenschäfte zu stellen sind. Außerdem sind die Wärmeeinflußzonen beiderseits der Schweißnaht beim Elektronenstrahlschweißen wesentlich schmaler als beispielsweise beim WIG-Schweißen (Wolfram-Inert-Gas). Auch der Nacharbeitsaufwand zur Glättung der Schweißnaht ist beim Elektronenstrahlschweißen gering.

Ist der Zusammengefügte Hohlschaft mit Durchbrüchen versehen, so kann der Hohlraum mit Polyethylen aufgefüllt werden. Diese Maßnahme ist erforderlich, da Durchbrüche dazu führen können, daß Knochen in den Hohlraum des Schaftes hineinwächst und ein derartiger absoluter Formschluß ein eventuell notwendiges Entfernen der Prothese erschwert. Die geringe Steifigkeit des Polyethylens hat nur geringen Einfluß auf form- und materialmäßig optimierte Eigenschaften der Hohlprothese. Um zu verhindern, daß das Polyethylen an den Durchbrüchen direkt mit dem Knochen in Berührung kommt, ist bevorzugt an den Durchbrüchen eine Apatitbeschichtung vorgesehen.

Weist der Prothesenschaft an seiner konvexen Außenseite Vertiefungen auf, ist deren Auffüllung mit einem Porocoatmaterial günstig.

Der Halsbereich der Prothese ist bevorzugt mit einem Steckkonus versehen, wodurch sich die Möglichkeit ergibt, Kugeln unterschiedlichen Durchmessers und mit verschiedenen Konusstecklängen zu verwenden.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 zwei nach einem vorteilhaften Verfahren hergestellte geschmiedete oder gepreßte Halbschalen der erfindungsgemäßen Endoprothese in perspektivischer Darstellung,
Figur 2 einen gemäß dem vorteilhaften Verfahren zusammengeschweißten Hohlschaft der erfindungsgemäßen Endoprothese mit Kopfteil in Seitenansicht,
Figur 3 eine Schnittdarstellung in Richtung A-A aus Figur 2,
Figur 4 eine Schnittdarstellung in Richtung B-B aus Figur 2 und
Figur 5 eine erfindungsgemäße Hohlschaftprothese mit entfernbarem Kopfteil - teilweise im Schnitt wiedergegeben.

Nachfolgend wird ein vorteilhaftes Ausführungsbeispiel eines vorteilhaften Verfahrens am Beispiel der Herstellung einer erfindungsgemäßen Hüftendoprothese erläutert.

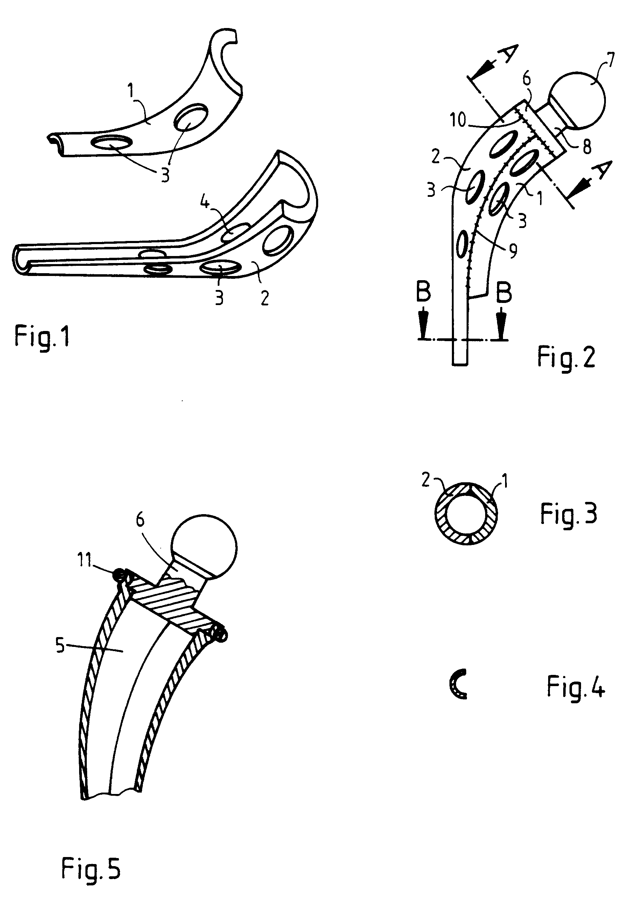

In einem ersten Verfahrensschritt werden zwei unterschiedliche, formmäßig aufeinander abgestimmte und zu einem Hohlschaft zusammenfügbare Halbschalen 1 und 2, wie in Figur 1 dargestellt, gepreßt oder geschmiedet. Als Werkstoff dient vorzugsweise eine Titan-Aluminium-Vanadium-Schmiedelegierung. Dieses Material ist nicht gießbar. Es besitzt aber viele in der Arthroplastik erwünschte Eigenschaften.

Die beiden Halbschalen 1 und 2 sind formmäßig so aufeinander abgestimmt, daß sie sich zu einem gebogenen Rohr mit stetig steigendem Durchmesser und im gleichen Richtungssinn stetig steigender Wanddicke zusammenfügen lassen. Die kontinuierliche Veränderung der Wanddicke in Achsrichtung des Schaftes wie auch die Einbringung bestimmter Wanddurchbrüche 3 bzw. Vertiefungen 4 in der inneren Oberfläche der beiden Halbschalen 1 und 2 ist bereits weitgehend beim Preß- bzw. Schmiedevorgang erzeugt worden. Derartige konstruktive Feinheiten dienen der Annäherung der Spannungsverläufe an die physiologische Festigkeit des umgebenden Knochenbereichs und seine Biegeeigenschaften.

Die Halbschalen 1 und 2 bilden den medialen, nach außen gebogenen Teil (Halbschale 1) und den lateralen, nach innen gebogenen Teil (Halbschale 2) des Hohlschaftes. Diese Teilung ist technologisch günstig, da der nach außen gebogene Teil am gelenkfernen Ende bevorzugt gegenüber dem nach innen gebogenen Teil verkürzt ist. (Insoweit wird auf die gleichzeitig eingereichte eine derartige Endoprothese betreffende Patentanmeldung derselben Anmelderin verwiesen.)

Nach dem Pressen oder Schmieden der beiden Halbschalen 1 und 2 und gegebenenfalls nach dem Erzeugen von Durchbrüchen und/oder Vertiefungen werden die Halbschalen 1 und 2 entgratet und geglättet. Ein eventuell nachfolgendes chemisches Abtragen oder ein spanabhebendes Verfahren zur Verbesserung der kontinuierlichen Dickenabnahme ist in dem noch "aufgeschnittenen" Zustand des Schaftes sowohl von innen als auch von außen günstig durchführbar.

Äußere Vertiefungen in der Wandung des Hohlschaftes 5 werden mit Porocoat- Material aufgefüllt, da ein hier eingewachsener Knochen leichter loszubrechen ist, als ein massiv in den Prothesenausschnitt eingewachsener Knochenbalken.

Eine weitere Variante, die Explantation zu ermöglichen, ist in Figur 5 dargestellt. Das Gelenkteil 6 wird nicht mit dem Hohlschaft verschweißt, sondern lösbar angeflanscht. Das kann vorteilhaft mittels eines Spannringes 11 mit Sollbruchstelle geschehen. Nach dem "Abbrechen" des Gelenkteiles 6 ist das Innere des Hohlschaftes 5 zugänglich und der Chirurg kann den evtl. in den Hohlraum hineingewachsenen Knochen mit Fräs- oder Bohrwerkzeugen entfernen ohne die Kortikalis zu beschädigen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Endoprothese mit Hohlschaft, insbesondere Hüftgelenkendoprothese, die aus in Längsrichtung geteilten Elementen durch Verschweißen der aneinandergrenzenden, im wesentlichen in Längsrichtung verlaufenden Nahtlinien zusammengefügt ist, **dadurch gekennzeichnet**,
daß die Elemente aus zwei durch Pressen oder Schmieden geformten Halbschalen bestehen, wobei die beiden Halbschalen (1 und 2) eine sich im wesentlichen kontinuierlich vom gelenknahen zum gelenkfernen Ende hin erstreckende Dickenabnahme aufweisen.

2. Endoprothese nach Anspruch 1, **dadurch gekennzeichnet**, daß die Halbschalen (1 und 2) aus einer Titan-Aluminium-Vanadium-Schmiedelegierung (TiAl6V4) bestehen.

3. Endoprothese nach einem der vorhergehenden Ansprüche, **dadurch gekennzeichnet**, daß die Halbschalen (1 und 2) mit Durchbrüchen (3) versehen sind bzw. vorhandene Durchbrüche (3) geglättet und entgratet sind.

4. Endoprothese nach einem der vorhergehenden Ansprüche, **dadurch gekennzeichnet**, daß der Hohlraum des Schaftes (5) mit Polyethylen aufgefüllt ist, wobei die Polyethylenoberflächen in den Durchbrüchen (3) mit Apatit beschichtet ist.

5. Endoprothese nach einem der vorhergehenden Ansprüche, **dadurch gekennzeichnet**, daß die konvexen Außenflächen der Halbschalen (1 und 2) mit Vertiefungen versehen sind, welche mit einem Porocoat-Material aufgefüllt sind.

6. Endoprothese nach einem der vorhergehenden Ansprüche, **dadurch gekennzeichnet**, daß die konkaven Innenflächen der Halbschalen (1 und 2) mit Vertiefungen (4) versehen sind.

7. Endoprothese nach einem der vorhergehenden Ansprüche, **dadurch gekennzeichnet**, daß ein Steckkonus (8) an das gelenknahe Ende des Hohlschaftes (5) angeschweißt ist.

8. Endoprothese nach einem der Ansprüche 1 bis 6, **dadurch gekennzeichnet**, daß ein Steckkonus (8) mittels eines eine Sollbruchstelle aufweisenden Klemmrings (11) mit dem gelenknahen Ende des Hohlschaftes (5) lösbar verbunden ist.

9. Verfahren zur Herstellung einer Endoprothese nach einem der vorhergehenden Ansprüche, **dadurch gekennzeichnet**,
daß zwei Halbschalen (1 und 2), die sich zu der Form des Schaftes der Prothese zusammenfügen lassen und dabei im Bereich zweier im wesentlichen in Längsrichtung verlaufender Nahtlinien aneinander grenzen, durch Pressen oder Schmieden erzeugt werden,
daß eine im wesentlichen kontinuierliche Dickenabnahme der Halbschalen (1 und 2) vom gelenknahen zum gelenkfernen Ende durch chemisches Abtragen oder spanabhebende Verformung erzeugt bzw. bei vorgepreßter oder vorgeschmiedeter Dickenabnahme nachbearbeitet wird, und
daß die beiden gepreßten oder geschmiedeten Halbschalen (1 und 2) im Bereich der Nahtlinien verschweißt werden.

10. Verfahren nach Anspruch 9, **dadurch gekennzeichnet**, daß die Durchbrüche (3) auf der Außenseite des Schaftes abgedeckt werden, die gelenkferne Öffnung des Schaftes verstopft wird, der Hohlraum des Schaftes (5) mit Polyethylen aufgefüllt wird, die Abdeckungen und der Stopfen entfernt werden und die Polyethylenoberfläche in den Durchbrüchen (3) mit Apatit beschichtet wird.

11. Verfahren nach einem der Ansprüche 9 und 10, **dadurch gekennzeichnet**, daß die Halbschalen (1 und 2) durch Elektronenstrahlschweißung miteinander verbunden werden.

12. Verfahren nach einem der Ansprüche 9 bis 11, **dadurch gekennzeichnet**, daß die Schweißnaht (9) zwischen den Halbschalen (1 und 2) und/oder die Schweißnaht (10) zwischen dem Steckkonus (8) und dem gelenknahen Ende des Hohlschaftes (5) manuell oder mit Hilfe eines Schleifgerätes geglättet werden.

## Claims

1. Endoprosthesis with hollow shaft, in particular hip-joint endoprosthesis, which is joined together from elements, divided in the longitudinal direction, by welding of the adjoining weld seam lines which extend essentially in the longitudinal direction, characterized in that the elements consist of two half shells which are formed by pressing or forging, the two half shells (1 and 2) exhibiting an essentially continuous decrease in thickness from the end near the joint to the end remote from the joint.

2. Endoprosthesis according to Claim 1, characterized in that the half shells (1 and 2) consist of a titanium/aluminium/vanadium forge alloy (TiAl6V4).

3. Endoprosthesis according to one of the preceding claims, characterized in that the half shells (1 and 2) are provided with openings (3), or existing openings (3) are smoothed and deburred.

4. Endoprosthesis according to one of the preceding claims, characterized in that the hollow cavity of the shaft (5) is filled with polyethylene, the polyethylene surfaces in the openings (3) being coated with apatite.

5. Endoprosthesis according to one of the preceding claims, characterized in that the convex outer surfaces of the half shells (1 and 2) are provided with depressions which are filled with a Porocoat material.

6. Endoprosthesis according to one of the preceding claims, characterized in that the concave inner surfaces of the half shells (1 and 2) are provided with depressions (4).

7. Endoprosthesis according to one of the preceding claims, characterized in that an attachment cone (8) is welded onto that end of the hollow shaft (5) near the joint.

8. Endoprosthesis according to one of Claims 1 to 6, characterized in that an attachment cone (8) is connected releasably to that end of the hollow shaft (5) near the joint by means of a clamping ring (11) which has a predetermined breaking point.

9. Method for producing an endoprosthesis according to one of the preceding claims, characterized in that two half shells (1 and 2), which can be joined together to give the shape of the shaft of the prosthesis and which in this respect adjoin one another in the area of two weld seam lines extending essentially in the longitudinal direction, are obtained by pressing or forging, in that an essentially continuous decrease in thickness of the half shells (1 and 2) from the end near the joint to the end remote from the joint is obtained by chemical milling or cutting work, or a pre-pressed or pre-forged decrease in thickness is finished, and in that the two pressed or forged half shells (1 and 2) are welded in the area of the weld seam lines.

10. Method according to Claim 9, characterized in that the openings (3) on the outer side of the shaft are covered, that opening of the shaft remote from the joint is plugged, the hollow cavity of the shaft (5) is filled with polyethylene, the coverings and the plug are removed, and the polyethylene surface in the openings (3) is coated with apatite.

11. Method according to either of Claims 9 and 10, characterized in that the half shells (1 and 2) are connected to one another by electron beam welding.

12. Method according to one of Claims 9 to 11, characterized in that the weld seam (9) between the half shells (1 and 2) and/or the weld seam (10) between the attachment cone (8) and that end of the hollow shaft (5) near the joint are smoothed manually or with the aid of a grinding device.

## Revendications

1. Endoprothèse a tige creuse, en particulier endoprothèse de l'articulation de la hanche, assemblée a partir d'éléments divisés en direction longitudinale par soudage des lignes de suture contiguës, courant essentiellement en direction longitudinale, caractérisée en ce que les éléments se composent de deux demi-capsules façonnées par emboutissage ou par forgeage, les deux demi-capsules (1 et 2) présentant une diminution d'épaisseur s'étendant essentiellement de façon continue de l'extrémité proche de l'articulation a l'extrémité éloignée de l'articulation.

2. Endoprothèse selon la revendication 1, caractérisée en ce que les demi-capsules (1 et 2) se composent d'un alliage de forge titane-aluminium-vanadium (TiAl₆V₄).

3. Endoprothèse selon l'une des revendications précédentes, caractérisée en ce que les demi-capsules (1 et 2) sont munies de perforations (3) ou selon les cas en ce que les perforations (3) présentes sont polies et ébarbées.

4. Endoprothèse selon l'une des revendications précédentes, caractérisée en ce que la cavité de l'arbre (5) est remplie de polyéthylène, les surfaces de polyéthylène dans les perforations (3) étant recouvertes d'apatite.

5. Endoprothèse selon l'une des revendications précédentes, caractérisées en ce que les surfaces externes convexes des demi-capsules (1 et 2) sont munies d'évidements qui sont remplis d'une matière de revêtement poreuse.

6. Endoprothèse selon l'une des revendications précédentes, caractérisée en ce que les surfaces internes concaves des demi-capsules (1 et 2) sont munies d'évidements (4).

7. Endoprothèse selon l'une des revendications précédentes, caractérisée en ce qu'un cône de raccordement (8) est soudé à l'extrémité de la tige creuse (5) proche de l'articulation.

8. Endoprothèse selon l'une des revendications 1 à 6, caractérisée en ce qu'un cône de raccordement (8) est relié de façon amovible à l'extrémité de la tige creuse (5) proche de l'articulation au moyen d'une bague de serrage (11) présentant un endroit de rupture prévue.

9. Procédé de fabrication d'une endoprothèse selon l'une des revendications précédentes, caractérisé
en ce qu'on produit par emboutissage ou forgeage deux demi-capsules (1 et 2), que l'on peut réunir pour obtenir la forme de la prothèse et qui sont alors contigües dans la zone de deux joints de suture courant essentiellement en direction longitudinale,
en ce qu'on produit une diminution essentiellement continue de l'épaisseur des demi-capsules (1 et 2) de l'extrémité proche de l'articulation à l'extrémité éloignée de l'articulation, par enlèvement chimique ou façonnage avec enlèvement de copeaux, ou selon les cas en ce qu'on effectue un post-usinage, avec diminution de l'épaisseur pré-emboutie ou pré-forgée, et
en ce qu'on soude les deux demi-capsules embouties ou forgées (1 et 2) dans la région du joint de suture.

10. Procédé selon la revendication 9, caractérisé en ce que les perforations (3) sur le côté extérieur de la tige sont recouvertes, en ce que l'ouverture de la tige éloignée de l'articulation est bouchée, en ce que la cavité de la tige (5) est remplie de polyéthylène, en ce qu'on retire les recouvrements et le bouchage et en ce qu'on recouvre la surface de polyéthylène dans les perforations (3) avec de l'apatite.

11. Procédé selon l'une des revendications 9 et 10, caractérisé en ce qu'on relie entre elles les demi-capsules (1 et 2) par soudage au moyen d'un rayon électronique.

12. Procédé selon l'une des revendications 9 à 11, caractérisé en ce qu'on polit à la main ou à l'aide d'un appareil de ponçage le joint de soudure (9) entre les demi-capsules (1 et 2) et/ou le joint de soudure (10) entre le cône de raccordement (8) et l'extrémité de la tige creuse (5).
